# EUROPÄISCHE PATENTSCHRIFT

(11) **EP 2 125 060 B1**
(45) Veröffentlichungstag und Bekanntmachung des Hinweises auf die Patenterteilung: **12.09.2012**
(21) Anmeldenummer: 08706775.7
(22) Anmeldetag: 21.01.2008
(51) Int. Cl.: A61L 29/08, A61L 29/16

(54) **VERFAHREN ZUR BELADUNG VON STRUKTURIERTEN OBERFLÄCHEN**
METHOD FOR LOADING STRUCTURED SURFACES
PROCÉDÉ DE CHARGE DE SURFACES STRUCTURÉES

(30) Priorität: 22.01.2007 DE 102007003184; 06.02.2007 US 899637 P
(43) Veröffentlichungstag der Anmeldung: 02.12.2009
(73) Patentinhaber: Eurocor Gmbh, 53227 Bonn (DE)
(72) Erfinder: ORLOWSKI, Michael, 53173 Bonn (DE)
(74) Vertreter: Arth, Hans-Lothar
(86) Internationale Anmeldenummer: PCT/DE2008/000095
(87) Internationale Veröffentlichungsnummer: WO 2008/089730

(56) Entgegenhaltungen:
- WO-A-00/32238
- WO-A-2004/028610
- WO-A-2008/061642
- US-A1- 2005 163 914
- US-A1- 2006 002 973

## Beschreibung

Die vorliegende Erfindung betrifft ein Verfahren zur Beladung von strukturierten Oberflächen aus vorzugsweise einem polymeren Material mit dem pharmakologischen Wirkstoff Paclitaxel, sowie die nach diesem Verfahren erhaltenen beschichteten Medizinprodukte.

Die Implantation von Gefäßstützen wie beispielsweise Stents ist heutzutage ein gängiger chirurgischer Eingriff zur Behandlung von Stenosen. Dabei ist noch immer eine sehr häufige Komplikation die sogenannte Restenose (recurrent stenosis), d.h. der Wiederverschluss des Gefäßes. Eine genaue begriffliche Beschreibung der Restenose ist in der Fachliteratur nicht aufzufinden. Die am häufigsten verwendete morphologische Definition der Restenose ist diejenige, die nach erfolgreicher PTA (perkutane transluminale Angioplastie) die Restenose als eine Reduktion des Gefäßdurchmessers auf weniger als 50% des normalen festlegt. Hierbei handelt es sich um einen empirisch festgelegten Wert, dessen hämodynamische Bedeutung und Beziehung zur klinischen Symptomatik einer soliden wissenschaftlichen Basis entbehrt. In der Praxis wird häufig die klinische Verschlechterung eines Patienten als Zeichen einer Restenose des vormals behandelten Gefäßabschnitts angesehen.

Die Restenose nach einer Stentimplantation ist eine der Hauptursachen für einen erneuten Krankenhausaufenthalt. Die während der Implantation des Stents verursachten Gefäßverletzungen rufen Entzündungsreaktionen hervor, die für den Heilungsprozeß in den ersten sieben Tagen eine entscheidende Rolle spielen. Ferner hat sich in jüngstes Vergangenheit auch herausgestellt, dass Stents, welche mit einer wirkstofffreisetzenden Beschichtung versehen sind, Spätthrombosen verursachen können, d.h. neben dem Problem der Restenose zudem noch ein Langzeitproblem nämlich das der Spätthrombosen besitzen.

Um diese Probleme zu vermeiden besteht auch die Möglichkeit, nur mittels eines beschichteten Katheterballons und ohne Stent ein sogenanntes "biological stenting" durchzuführen, d.h. die Gefäßaufweitung an der verengten Stelle mittels Dilatation eines beschichteten Katheterballons vorzunehmen, wobei während einer kurzen Dilatationszeit des Katheterballons genügend pharmakologischer Wirkstoff auf die Gefäßwand übertragen wird, dass aufgrund der Gefäßaufweitung und der Wirkstoffübertragung eine erneute Verengung oder eine erneuter Verschluß des Gefäßes unterbleibt.

Solche beschichteten Katheterballons sind bereits aus WO2005/089855 A1 bekannt und die internationale Patentanmeldung WO 2004/028582 A1 offenbart Faltenballons, welche insbesondere in den Falten mit einer Zusammensetzung aus einem pharmakologischen Wirkstoff und einem Kontrastmittel beschichtet werden. Ein Sprühbeschichtungsverfahren für Katheterballons ist in WO 2004/006976 A1 beschrieben.

WO 2004/028610 offenbart eine Methode zur Beschichtung von Katheterballons mit Paclitaxel umfassend: Die Bereitstellung eines Katheterballons und einer Lösung von Paclitaxel in einem Lösungsmittel, der anschliessenden Beschichtung der Oberfläche des Katheterballons mit dieser Lösung und dem abschliessenden Schritt des Trocknens der Beschichtung. Auch WO 00/32238 offenbart ein Verfahren zur Beschichtung von Gefässendoprothesen und Ballonkathetern. US 2006/0002973 beschreibt ein Verfahren zum Beschichten eines Katheterballons mit einer polymeren Schicht, in die anschliessend Paclitaxel eingebracht wird. US 2005/0163914 beschreibt ein Verfahren zum Beschichten von Endoprothesen, z.B. Stents, mit Wirkstoffen in einer polymeren Schicht. Dabei wird der Wirkstoff in einem organischen Lösungsmittel gelöst und auf die polymere Schicht der Stentoberfläche aufgetragen. Anschliessend wird durch die Zugabe von Wasser der Arzneistoff ausgefallt.

Da sich der Wirkstoff Paclitaxel als besonders geeignet für die Verhinderung von Restenose herausgestellt hat, wie insbesondere dem europäischen Patent Nr. EP 0 706 376 B1 zu entnehmen ist, jedoch beschichtete Stents die oben beschriebenen Nachteile hinsichtlich Spätthrombosen besitzen, hat sich die Aufgabe gestellt, den Wirkstoff Paclitaxel so auf einen Katheterballon aufzubringen, dass eine Beschichtung entsteht, welche sich leicht von dem Ballon ablöst und effektiv auf die Gefäßwand übertragen werden kann.

Diese Aufgabe wird durch die technische Lehre der unabhängigen Patentansprüche gelöst. Weitere vorteilhafte Ausgestaltungen der Erfindung ergeben sich aus den abhängigen Ansprüchen, der Beschreibung und den Beispielen.

Überraschend wurde gefunden, dass ein Beschichtungsverfahren der folgenden Art die gestellte Aufgabe besonders gut löst.

Dieses Verfahren zur Beladung oder Beschichtung von dilatierbaren Katheterballons umfasst die folgenden Schritte:
I) Bereitstellung eines dilatierbaren Katheterballons,
II) Bereitstellung einer Lösung von Paclitaxel in Dimethylsulfoxid,
III) Durchführung einer Strukturierung der Oberfläche des dilatierbaren Katheterballons,
IV) Benetzung der Oberfläche des dilatierbaren Katheterballons mit der Lösung von Paclitaxel in Dimethylsulfoxid,
V) Zugabe von Wasser, wobei das Wasser einen pH-Wert von 3 bis 5 aufweist, welches in der Lage ist Paclitaxel auszufällen, auf die mit der Lösung von Paclitaxel in Dimethylsulfoxid benetzte Oberfläche des dilatierbaren Katheterballons,
VI) Trocknung der benetzten Oberfläche des dilatierbaren Katheterballons.

Als dilatierbare Katheterballons können beliebige kommerziell erhältliche Katheterballons eingesetzt werden. Vorzugsweise werden jedoch sogenannte Faltenballons verwendet, wie sie beispielsweise in der internationalen

Patentanmeldung WO 94/23787 A1 von David H. Rammler, Labintelligence, U.S.A. oder der internationalen Patentanmeldung WO 03/059430 A1 der Scimed Life Sciences, Inc., U.S.A. oder der internationalen Patentanmeldung WO 2004/028582 A1 von Herrn Prof. Dr. Ulrich Speck oder dem europäischen Patent Nr. EP 0519063 B1 der Medtronic Inc., U.S.A. beschrieben sind.

Derartige Ballons weisen Falten oder Flügel auf, welche im komprimierten Zustand weitgehend geschlossene Hohlräume bilden, welche sich bei der Dilatation nach außen wölben und in den Falten befindliche Stoffe freisetzen bzw. gegen die Gefäßwand drücken können.

Derartige Ballons haben den Vorteil, dass die in den Falten eingeschlossenen Substanzen bzw. das in den Falten eingeschlossene Paclitaxel während des Einführens des Katheters vor der zu frühen Ablösung geschützt ist.

Um den Wirkstoff Paclitaxel vor dem vorzeitigen Ablösen vom Katheterballon zu schützen, kann Paclitaxel auch in eine Trägersubstanz vorzugsweise einen polymeren Träger eingelagert oder eingebettet werden. Um diesen Träger bzw. polymeren Träger enthaltend Paclitaxel auf den Katheterballon aufzubringen, wird die Trägersubstanz der Lösung aus DMSO und Paclitaxel zugesetzt. Solche DMSO-Lösungen enthaltend Paclitaxel und die Trägersubstanz werden dann mit den üblichen Verfahren insbesondere Sprüh- oder Tauchverfahren auf den Katheterballon aufgebracht. Als Träger eignen sich die auch als Ballonmaterial verwendbaren Stoffe insbesondere polymere als auch polymerisierbare Stoffe wie weiter unter aufgeführt.

Auch in solchen Fällen, wo die Beschichtung, d.h. das Paclitaxel nicht durch die Falten eines Faltenballons geschützt wird oder das Paclitaxel nicht in einem Träger eingelagert wird, kann eine solche Menge an reinem Wirkstoff Paclitaxel auf den Katheterballon aufgetragen werden, dass auch mit einer kalkulierten vorzeitig während des Einführens des Katheterballons abgelösten Menge an Paclitaxel von ca. 30 % der Gesamtmenge, noch eine genügend große therapeutisch wirkende Menge an Paclitaxel auf dem Ballon vorhanden ist, wenn dieser sein Ziel erreicht hat und welche dann bei der Dilatation auf die Gefäßwand übertragen werden kann.

Somit ist eine Schutz des Wirkstoffs Paclitaxel vor frühzeitigem Ablösen z.B. durch Einbringung unter die Falten oder durch Einlagerung in einen Träger auf der Oberfläche des Katheterballons bevorzugt jedoch nicht zwingend erforderlich.

Die Faltenballons werden erfindungsgemäß im expandierten Zustand beschichtet, so dass sich für die Beschichtung von Faltenballons ein etwas modifiziertes Verfahren ergibt, welches die folgenden Schritte umfasst:
I) Bereitstellung eines dilatierbaren Katheterballons,
IIa) Bereitstellung einer Lösung von Paclitaxel in Dimethylsulfoxid,
IIb) Expansion des Faltenballons so, dass die Falteninnenräume zugänglich werden,
III) Durchführung einer Strukturierung der Oberfläche des dilatierbaren expandierten Katheterballons,
IV) Benetzung der Oberfläche des dilatierbaren Katheterballons mit der Lösung von Paclitaxel in Dimethylsulfoxid,
V) Zugabe von Wasser, wobei das Wasser einen pH-Wert von 3 bis 5 aufweist, welches in der Lage ist Paclitaxel auszufällen, auf die mit der Lösung von Paclitaxel in Dimethylsulfoxid benetzte Oberfläche des dilatierbaren Katheterballons,
VI) Trocknung der benetzten Oberfläche des dilatierbaren Katheterballons,
VII) Rückfaltung des Faltenballons in den komprimierten Zustand.

Die Verfahren zur Beladung oder zur Beschichtung von Katheterballons als auch von Faltenballons wird in der Regel in der Reihenfolge der Schritte I) bis VI) bzw. I) bis VII) durchgeführt, wobei die Schritte I) und II) in ihrer Reihenfolge natürlich auch vertauscht werden können.

Ferner können bei den erfindungsgemäßen Beschichtungsverfahren die Schritte IV) bis VI) auch mehrmals wiederholt werden. Schritt V) umfassend die Aufbringung eines Lösungsmittels, welches in der Lage ist Paclitaxel auszufällen muss mindestens einmal ausgeführt werden, braucht aber nicht zwingend bei einem weiteren Beschichtungsvorgang wiederholt zu werden. Daher besteht auch die Möglichkeit, dass beim ersten Beschichtungsvorgang die Schritte IV) bis VI), d.h., die Schritt I) bis VI) durchlaufen werden und bei einer Wiederholung des Beschichtungsvorgangs nur noch die Schritte IV) bis VI) oder auch nur noch die Schritte IV) und VI) wiederholt werden.

In der Regel wird der Beschichtungsvorgang einmal oder zweimal oder dreimal wiederholt, wobei dies jedoch nicht zwingend ist. Auch ein einmaliger Beschichtungsvorgang kann ausreichen, um die erforderliche Menge an Paclitaxel auf den Katheterballon aufzutragen.

In der Regel wird eine Menge von 0,5 µg bis 50 µg Paclitaxel pro mm² Oberfläche des zu beschichtenden Ballonkatheters und bevorzugt eine Menge von 1 µg bis 20 µg Paclitaxel pro mm² Oberfläche des zu beschichtenden Ballonkatheters auf die Ballonoberfläche aufgetragen. Pro Katheterballon werden vorzugsweise 10 bis 1000 µg Paclitaxel und insbesondere bevorzugt werden pro Katheterballon 20 µg bis 400 µg auf den Ballon aufgetragen.

Paclitaxel kann kommerziell von mehreren Anbietern bezogen werden. Paclitaxel ist unter dem Markennamen Taxol^{®} bekannt und besitzt noch diverse Synonyme wie beispielsweise

BMS 181339-01, BMS-181339, BMS-181339-01, Capxol, DRG-0190, DTS-301, Ebetaxel, Genaxol, Genexol, Genexol-PM, HSDB 6839, Intaxel, KBio2_002509, KBio2_005077, KBio2_007645, KBio3_002987, KBioGR_002509, KBioSS_002517, LipoPac, MBT 0206, MPI-5018, Nanotaxel, NCI60-000601, Nova-12005, NSC 125973, NSC-125973, NSC125973, Onxol, Pacligel, Paxceed, Paxene, Paxoral, Plaxicel, QW 8184, SDP-013, TA1, Tax-11-en-9-one, TaxAlbin, Taxol A, Xorane oder Yewtaxan.

Die chemische Struktur ist wie folgt:

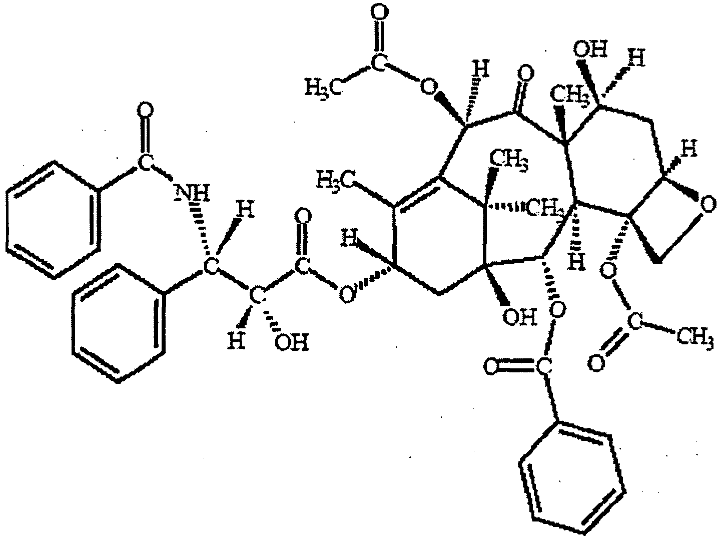

Die IUPAC-Nomenklatur lautet: [2aR-[2a,4,4a,6,9(R*,S*),11,12,12a,12b]]-(Benzoylamino)-hydroxybenzolpropionsäure-6,12b-bis-(acetyloxy)-12-(benzoyloxy)-2a-3, 4, 4a, 5, 6, 9, 10, 11, 12, 12a, 12b-dodecahydro-4,11-dihydroxy-4a,8,13,13-tetramethyl-5-oxo-7,11-methano-1H-cyclodeca[3,4]benz[1,2-b]oxet-9-yl-ester).

Paclitaxel ist in Dimethylsulfoxid (DMSO) und Methanol sowie wasserfreiem Ethanol gut löslich, jedoch in Wasser nur relativ schlecht löslich. Insbesondere bei einem pH-Wert zwischen 3 und 5 ist Paclitaxel besonders stabil und lange lagerfähig, wohingegen es bei alkalischen pH-Werten relativ instabil ist.

Als Lösungsmittel für Paclitaxel wird Dimethylsulfoxid (DMSO) verwendet. DSMO löst zum einen die erforderlichen Mengen an Paclitaxel und greift das Material des Ballonkatheters nur geringfügig an. Als Materialien für Ballonkatheter werden die weiter unter aufgeführten verwendet, wobei folgende Polymere insbesondere bevorzugt sind: Polyamide, Polyamid-Polyether-Polyester-Blockcopolymere, Polyurethane, Polyester sowie Polyolefine.

Ferner hat DMSO die Eigenschaft, dass durch sukzessive Erhöhung des Wassergehalts die Löslichkeit von Paclitaxel in DMSO langsam verringert werden kann bis hin zu einem Punkt, wo Paclitaxel beginnt auszufallen bzw. auszukristallisieren. Die Struktur des ausgefallenen Paclitaxels ist unklar, jedoch hat sich gezeigt, dass eine gemäß dem erfindungsgemäßen Verfahren erhaltene Paclitaxelbeschichtung sich besonders gut von dem Katheterballon ablöst und auf die Gefäßwand übertragen werden kann.

In dem Dimethylsulfoxid ist Paclitaxel in Mengen bis 150 mg / ml löslich. Vorzugsweise werden in einem ml DMSO 1 mg - 150 mg, weiter bevorzugt 10 mg bis 90 mg und insbesondere bevorzugt 40 mg - 60 mg Paclitaxel gelöst.

Ferner wird vorzugsweise getrocknetes, d.h. weitgehend wasserfreies DMSO verwendet. Der Wassergehalt des DMSO sollte 5 Vol.-%, vorzugsweise 3 Vol.-% und insbesondere bevorzugt 1 Vol.-% nicht überschreiten.

Wie bereits erwähnt, kann der DMSO-Lösung ein Träger für den Wirkstoff Paclitaxel zugesetzt werden. Bei dem Träger handelt es sich vorzugsweise um ein biostabiles oder biologisch abbaubares Polymer, vorzugsweise ausgewählt aus der Gruppe von Polymeren, welche weiter unten als Materialien für den Katheterballon offenbart sind. Der oder die verwendeten Trägersubstanzen können einen Gewichtsanteil von bis zu 70 Gew.-%, bevorzugt von bis zu 50 Gew.-% und insbesondere bevorzugt von bis zu 30 Gew.-% bezogen auf das Gewicht der gesamten Lösung ausmachen.

Gemäß den erfindungsgemäßen Verfahren wird zwecks Verbesserung der Anhaftung des Paclitaxels und zur Unterstützung der Ausfällung oder der Kristallisation des Paclitaxels die Oberfläche des Katheterballons mechanisch, chemisch, elektronisch und/oder mittels Strahlung strukturiert.

Die Strukturierung der Oberfläche des Katheterballons hat das Ziel, im Nanometerbereich bis hin zum Mikrometerbereich die Oberfläche des Katheterballons zu verändern also eine Art mikroraue Oberflächenstruktur zu schaffen. Die Oberflächenstrukturierung erfolgt vorzugsweise über den gesamten zu beschichtenden Bereich des Katheterballons und kann zu geordneten Strukturen oder zufallsmäßigen Strukturen führen.

Die Katheterballons können aus folgenden Materialien bestehen:
Parylen C, Parylen D, Parylen N, Parylen F, Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride, Polymaleinsäureanhydride, Polyhydroxymethacrylate, Fibrin, Polycyanoacrylate, Polycaprolactondimethylacrylate, Poly-β-Maleinsäure Polycaprolactonbutylacrylate, Multiblockpolymere aus Oligocaprolactondiole und Oligodioxanondiole, Polyetherestermultiblockpolymere aus PEG und Polybutylenterephtalat, Polypivotolactone, Polyglycolsäuretrimethylcarbonate Polycaprolactonglycolide, Poly(γ-ethylglutamat), Poly(DTH-Iminocarbonat), Poly(DTE-co-DT-carbonat), Poly(Bisphenol A-iminocarbonat), Polyorthoester, Polyglycolsäuretrimethylcarbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxidpropylenoxid, weiche Polyurethane, Polyurethane mit Aminosäurereste im Backbone, Polyetherester, Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, Polyhydroxyalkanoate, Pectinsäure, Actinsäure, Carboxymethylsulfat, Albumin, Hyaluronsäure, Chitosan und seine Derivate, Heparansulfate und seine Derivate, Heparine, Chondroitinsulfat, Dextran, ß-Cyclodextrine, Copolymere mit PEG und Polypropylenglycol, Gummi arabicum, Guar, Gelatine, Collagen Collagen-N-Hydroxysuccinimid, Lipide, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane, Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone, Polysiloxane, Polydimethylsiloxane, Polyvinylhalogene und Copolymere, Celluloseether, Cellulosetriacetate, Chitosane und Copolymere und/oder Mischungen der vorgenannten Polymere.

Bevorzugt sind jedoch Polyamide, Polyamid-Polyether-Polyester-Blockcopolymere, Polyurethane, Polyester sowie Polyolefine.

Wichtig ist, dass die Katheterballons bei der Strukturierung der Ballonoberfläche nicht beschädigt werden oder deren Expansionsfähigkeit nachteilig beeinflusst wird. Somit dürfen die Verfahren zur Mikrostrukturierung der Ballonoberfläche keine Löcher, Mikroporen oder Risse in dem Ballonmaterial verursachen. Idealerweise wird wirklich nur die äußere Oberfläche der Ballons bis z einer Tiefe von maximal 1 µm strukturiert.

Die Strukturierung des dilatierbaren Katheterballons kann mechanisch durch eine reibenartige Vorrichtung, eine Reibe oder durch ein Strahlverfahren mit festen Teilchen wie beispielsweise einem Sandstrahlverfahren erfolgen.

Ein chemisch-mechanisches Verfahren verwendet eine Aufschlämmung oder Dispersion von festen Teilchen in einem Lösungsmittel insbesondere Wasser. Derartige Verfahren sind auch als chemische Polierverfahren bekannt. Ein Verreiben solcher Zusammensetzungen auf der Oberfläche des Ballonmaterials macht dieses rau ohne zu tiefen Rissen oder Löchern zu führen.

Bei einem rein chemischen Strukturierungsverfahren werden Säuren, Basen, ätzende Chemikalien und/oder oxidierende Chemikalien eingesetzt, welche die Oberfläche das Ballonmaterials angreifen. Derartige Chemikalien sind jedoch mit Vorsicht einzusetzen, da das Ballonmaterial bei zu langer oder zu intensiver Einwirkung beschädigt werden könnte.

Bei einem elektrischen oder elektronischen Verfahren zur Strukturierung der Oberfläche des dilatierbaren Katheterballons erfolgt die Strukturierung mittels aufgrund von Stromfluss erwärmter Leiter. Eine feine warme, heiße oder glühende Nadel kann beispielsweise eingesetzt werden, um das Ballonmaterial auf seiner Oberfläche aufzuschmelzen, wodurch auf der Oberfläche bestimmte Muster insbesondere bei der Bewegung der Nadel über die Oberfläche des Katheterballons erzeugt werden können.

Ein elegantes Verfahren zur Erzeugung geordneter Strukturen insbesondere in Form von Mikrovertiefungen oder Mikrokanälen kann durch die Verwendung von Lasern oder grundsätzlich von stark fokussierter Strahlung erreicht werden. Diese arbeiten sehr präzise und können insbesondere zur Erzeugung definierter Strukturen wie Gittern, Spiralen oder Linien eingesetzt werden.

Eine solche Oberflächenstrukturierung der Katheterballonoberfläche führt zu einer Vergrößerung der Oberfläche und damit zu einer möglichen höheren Wirkstoffbeladung im Vergleich zu einer unstrukturierten Ballonoberfläche. Die Mikrostrukturierung schafft somit eine dreidimensionale Oberfläche, welche mit einer größeren Menge Paclitaxel beladen werden kann. Ferner findet im Vergleich zur glatten unbehandelten Ballonoberfläche eine bessere Adhäsion des Wirkstoffs statt, der durch die Strukturierung auch besser gegen das Abwaschen oder Ablösen im Blutstrom geschützt ist, da er zumindest teilweise in den gebildeten Poren eingelagert ist.

Die Benetzung der strukturierten oder mikro- bis nanomodifizierten Oberfläche des Katheterballons kann mittels aller gängiger Verfahren geschehen. Die Lösung aus Paclitaxel in DMSO kann beispielsweise mittels Sprühverfahren, Tauchverfahren, Plasmaabscheidungsverfahren, Streichverfahren oder Spritzverfahren auf die Ballonoberfläche aufgetragen werden. Während bei dem Tauchverfahren und Plasmaabscheidungsverfahren zumeist die gesamte Oberfläche des Katheterballons beschichtet wird, können Sprühverfahren, Streichverfahren als auch Spritzverfahren dazu eingesetzt werden, nur einen Teilbereich der Ballonoberfläche zu beschichten.

Erfindungsgemäß ist nicht erforderlich, dass der Katheterballon vollständig beschichtet wird. Auch eine teilweise Beschichtung oder die Beladung bestimmter Strukturelemente auf der Oberfläche des Katheterballons kann ausreichen. Einen speziellen Katheterballon mit Mikronadeln oder Mikroporen oder Mikrokammern offenbart die internationale Patentanmeldung Nr. WO 02/043796 A2 der Scimed Life Systems, Inc., U.S.A., wobei befüllbare und strukturierte Bereiche auf der Ballonoberfläche existieren. Bei dieser Ausführungsform würde die Beladung oder Befüllung von Teilbereichen der Ballonoberfläche ausreichen, um den erwünschten therapeutischen Erfolg zu erzielen, wobei selbstverständlich auch die gesamte Oberflächen beschichtet werden kann.

Ferner besteht natürlich auch die Möglichkeit, den Katheterballon teilweise in bestimmten Abschnitten zu beschichten und sukzessive weitere Bereiche zu beschichten bis man falls erwünscht, letztendlich einen vollständig beschichteten Katheterballon erhält.

Nach der vollständigen oder teilweisen Benetzung der Oberfläche des Katheterballons hat sich herausgestellt, dass durch reine Verdunstung des Lösungsmittels nicht die gewünschte Beschichtung entsteht. Somit ist insbesondere der Schritt V) der erfindungsgemäßen Beschichtungsverfahren essentiell, wo der benetzen Oberfläche unmittelbar nach der Benetzung oder unmittelbar nach dem Auftragen oder Aufbringen der Lösung von Paclitaxel in DMSO und bevor das DSMO zu 50%, bevorzugt zu 25% und insbesondere bevorzugt zu 10% verdunstet ist, Wasser aufgebracht wird, welches in der Lage ist, Paclitaxel auszufällen.

Diese wasser wird zu dem DSMO gegeben und soll die Löslichkeit von Paclitaxel in DSMO verringern. Diese wasser hat die Eigenschaft, dass sich Paclitaxel darin nicht oder nur schlecht löst, so dass bei dem Zusatz diese wasser zum DMSO das Löslichkeitsprodukt von Paclitaxel in DSMO herabgesetzt wird.

Dieses Wasser mit einem pH-Wert van 3 bis 5 dient zum Ausfällen des Paclitaxel und wird bevorzugt homogen in dem DMSO verteilt. Es wird so viel Fällungsmittel vorzugsweise im Sprühverfahren oder im Spritzverfahren oder im Pipettierverfahren zugesetzt, bis sich eine leichte Trübung ergibt oder bis Paclitaxel erkennbar beginnt auszufallen. An diesem Punkt kann noch ein wenig Fällungslösungsmittel zugesetzt werden oder man lässt das Lösungsmittelgemisch aus DMSO und Fällungslösungsmittel verdunsten. Selbstverständlich kann die Trocknung der Beschichtung auch durch Anlagen von Unterdruck oder Vakuum beschleunigt werden, jedoch ist eine Trocknung an der Lust zu bevorzugen.

Dadurch wird die erfindungsgemäße und schwer charakterisierbare Paclitaxelbeschichtung erhalten.

Wasser ist als Fällungslösungsmittel geeignet, da sich darin Paclitaxel nur sehr schlecht löst und Wasser zudem physiologisch unbedenklich als auch unbedenklich für das Ballonmaterial ist.

Am besten wird destilliertes, entsalztes oder deionisiertes Wasser eingesetzt, welches mittels üblicher Ionenaustauschchromatographie erhalten werden kann.

Zudem weist dass Wasser einen pH-Wert im Bereich von 3 bis 5,und insbesondere bevorzugt im Bereich von 3,5 bis 4,5, auf. Der pH-Wert kann mittels organsicher Säuren und Salzen dieser organischen Säuren eingestellt werden, wobei grundsätzlich möglichst wenig Puffer in Form von Säuren und konjugierten Basen eingesetzt werden sollten.

Als Säuren für die Einstellung des pH-Wertes eignen sich insbesondere Ameisensäure, Essigsäure, Propionsäure, Oxalsäure, Salicylsäure, Weinsäure, Fumarsäure, Gluconsäure, Milchsäure, Äpfelsäure, Ascorbinsäure, Maleinsäure, Malonsäure, Hydroxymaleinsäure, Brenztraubensäure, Phenylessigsäure, Benzoesäure, Glutarsäure, Camphersulfonsäure oder Chinasäure (Chininsäure). Falls erforderlich können zudem Salze dieser Säuren wie beispielsweise das Natriumacetat, Calciumoxalat oder Lithiummalonat eingesetzt werden.

Ferner kann der Lösung aus Paclitaxel in DMSO auch noch ein weiterer Wirkstoff zugesetzt werden, der beispielsweise aus folgender Gruppe ausgewählt werden kann:
Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren und ihre Derivate, Bruceanole A,B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, Mitoxanthrone, Mycophenolatmofetil, ß-Lapachon, Podophyllotoxin, Podophyllsäure-2-ethylhydrazid, Molgramostim (rhuGM-CSF), Peginterferon α-2b, Lanograstim (r-HuG-CSF), Macrogol, Selectin (Cytokinantagonist), Cytokininhibitoren, COX-2-Inhibitor, Angiopeptin, monoklonale Antikörper, die die Muskelzellproliferation hemmen, bFGF-Antagonisten, Probucol, Prostaglandine, 1-Hydroxy-11-Methoxycanthin-6-on, Scopolectin, NO-Donoren, Pentaerythrityltetranitrat und Syndnoeimine, S-Nitrosoderivate, Tamoxifen, Staurosporin, ß-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel und dessen Derivate, 6-α-Hydroxy-Paclitaxel, Taxotere, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, β-Sitosterin, Myrtecain, Polidocanol, Nonivamid, Levomenthol, Ellipticin, D-24851 (Calbiochem), Colcemid, Cytochalasin A-E, Indanocine, Nocadazole, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA₋ und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika, Cefadroxil, Cefazolin, Cefaclor, Cefotixin Tobramycin, Gentamycin, Penicilline, Dicloxacillin, Oxacillin, Sulfonamide, Metronidazol, Enoxoparin, Heparin, Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioproteaseinhibitoren, Prostacyclin, Vapiprost, Interferon α, β und γ, Histaminantagonisten, Serotoninblocker, Apoptoseinhibitoren, Apoptoseregulatoren, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide, Inotodiol, Maquirosid A, Ghalakinosid, Mansonin, Streblosid, Hydrocortison, Betamethason, Dexamethason, nichtsteroidale Substanzen (NSAIDS), Fenoporfen, Ibuprofen, Indomethacin, Naproxen, Phenylbutazon, antivirale Agentien, Acyclovir, Ganciclovir, Zidovudin, Clotrimazol, Flucytosin, Griseofulvin, Ketoconazol, Miconazol, Nystatin, Terbinafin, antiprozoale Agentien, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin.

Ferner betrifft die vorliegende Erfindung Katheterballons und insbesondere Faltenballons für Katheter, welche nach einem erfindungsgemäßen Verfahren beschichtet worden sind.

Die Katheterballons weisen eine Beschichtung aus vorrangig reinem Paclitaxel auf. Somit tragen die Katheterballons eine reine Wirkstoffschicht aus Paclitaxel, wobei in dieser Schicht nur noch Spuren von DMSO und eventuell einem anderen Lösungsmittel und minimale Rückstände der verwendeten Säure und eventuell deren konjugierter Base enthalten sind.

Aufgrund des erfindungsgemäßen Beschichtungsverfahrens besitzt das auf der Oberfläche des Katheterballons getrocknete Paclitaxel eine besondere Beschaffenheit, welche schwer zu charakterisieren ist, jedoch für die Übertragung auf die Zellwand und die Aufnahme in vor allem die glatten Muskelzellen essentiell zu sein scheint.

Bei Faltenballons befindet sich ein Teil der Paclitaxel-Beschichtung im komprimierten Zustand unter den Falten. Diese Menge reicht aus, um den gewünschten therapeutischen Erfolg zu erzielen, auch wenn die restliche freiliegende Ballonoberfläche nicht mit Wirkstoff beschichtet ist.

Somit betrifft die vorliegende Erfindung auch Katheter umfassend einen Katheterballon, der erfindungsgemäß beschichtet ist.

Derartige Katheter werden vorzugsweise zur Behandlung verengter Gefäßsegmente insbesondere von Blutgefäßen und zur Behandlung und Prophylaxe von Stenose, Restenose, Arteriosklerose, Atherosklerose sowie fibrotischer Gefäßverengung eingesetzt.

Ferner eignen sich die erfindungsgemäß beschichteten Katheterballons insbesondere zur Behandlung und/oder Prophylaxe von In-Stent-Restenose, also von einer erneuten Gefäßverengung innerhalb eines bereits implantierten Stents, wo eine weitere Stentplatzierung innerhalb eines bereits implantierten Stents sehr problematisch bis medizinisch nicht durchführbar ist. Mittels der Wirkstoffapplikation mit Hilfe eines erfindungsgemäß beschichteten Katheters bzw. erfindungsgemäß beschichteten Katheterballons eines Dilatatidnskatheters kann eine solche In-Stent-Restenose effektiv und ohne Platzierung eines weiteren Stents behandelt werden.

Ferner sind die erfindungsgemäß beschichteten Katheterballons insbesondere geeignet, kleine Gefäße zu behandeln, vorzugsweise solche mit einem Gefäßdurchmesser kleiner 2,25 mm.

Bevorzugt ist der Einsatz der erfindungsgemäß beschichteten Katheterballons im kardiovaskulären Bereich, wobei die erfindungsgemäß beschichteten Katheterballons sich auch zur Behandlung von Gefäßverengungen von Gallenwegen, Speiseröhre, Harnwegen, Bauchspeicheldrüse, Nierenwege, Lungenwege, Luftröhre, Dünndarm sowie Dickdarm eignen.

Es besteht jedoch auch die Möglichkeit auf den erfindungsgemäßen Katheterballon einen beschichteten oder einen unbeschichteten Stent zu krimpen, d.h. aufzusetzen und gleichzeitig mit der Wirkstoffabgabe an die Gefäßwand während der Dilatation des Katheterballons auch einen Stent zu setzen.

Die folgenden Beispiele stellen mögliche Ausführungsformen der vorliegenden Erfindung dar, ohne jedoch den Schutzumfang auf diese konkreten Beispiele beschränken zu wollen.

### Beispiele

### Beispiel 1:

Ein kommerziell erhältlicher Dilatationskatheter mit expandierbarem Ballon aus einem Polyamid wird bereitgestellt.

Mittels eines Sandstrahlverfahrens wird die Oberfläche des Katheterballons im Nanometer- bis Mikrometerbereich aufgeraut.

In getrocknetem Dimethylsulfoxid (DMSO) wird Paclitaxel (kommerziell erhältlich von Sigma, Fermentek, BC Biotech oder Arianna International) in einer Konzentration von 50 mg pro ml DMSO gelöst.

Die Lösung von Paclitaxel in DMSO wird auf den Katheterballon gesprüht und anschließend wird ein feiner Nebel von Wasser mit einem pH-Wert von 3,5 auf die mit der Lösung aus Paclitaxel in DMSO benetzte Oberfläche des Katheterballons gesprüht. Das Wasser wurde vorher destilliert und danach mittels Essigsäure und Natriumacetat auf einen pH-Wert von 3,5 eingestellt. Der Wassernebel wird so lange auf die benetzte Oberfläche gesprüht, bis das Paclitaxel beginnt auszufallen.

Nun lässt man die Oberfläche des Katheterballon an der Luft trocknen und wiederholt den Beschichtungsvorgang mit Paclitaxel in DMSO und die anschließende Ausfällung mittels Wassernebel noch weitere zwei Mal.

Die Menge an Paclitaxel auf dem Katheterballon beträgt 3 µg pro mm² Ballonoberfläche.

Danach wird der Katheterballon getrocknet, sterilisiert und der gesamte Katheter verpackt.

### Beispiel 2:

Ein Faltenballon wird bereitgestellt, wie er beispielsweise in WO 2004/028582 A1, WO 94/23787 A1 oder WO 03/059430 A1 beschrieben ist. Der Faltenballon besitzt insgesamt 5 Falten, welche im komprimierten Zustand des Ballons einen Hohlraum einschließen und sich im expandierten Zustand nach außen wölben, so dass der Ballon im expandierten Zustand weitgehend eine rohrförmige Gestalt annimmt.

Der Faltenballon wird expandiert und danach seine Oberfläche mittels eines sogenannten "chemical polishing"-Verfahrens aufgeraut, wobei bei diesem Verfahren eine Aufschlämmung von feinen Partikeln im vorzugsweise Mikrometerbereich eingesetzt wird und diese Aufschlämmung auf der Oberfläche des expandierten Katheterballons derart verrieben wird, dass eine aufgeraute Oberfläche entsteht.

Eine Lösung von 80 mg Paclitaxel in 1,0 ml DMSO und 0,1 ml Methanol wird angesetzt und so lange gerührt, bis sich das gesamte Paclitaxel aufgelöst hat.

Der aufgeraute expandierte Katheterballon wird in diese Lösung aus Paclitaxel in DMSO und Methanol getaucht und während der Ballon rotiert mit einem Neben aus Wasser besprüht, wobei das Wasser vorher mittels Ionenaustauscher deionisiert und danach mit möglichst wenig Oxalsäure und Calciumoxalat auf einen pH-Wert von 4.0 eingestellt worden ist.

Sobald die ersten feinen Paclitaxelkristalle sich bilden, lässt man den Katheterballon an der Luft langsam trocknen, so dass sich ein feiner und weitgehend gleichmäßiger Paclitaxelbelag auf dem Katheterballon ausbildet. Nach der vollständigen Trocknung wird der erstmalig beschichtete Katheterballon weitere drei Mal in die Paclitaxellösung getaucht und danach getrocknet, ohne ihn erneut mit einem Wassernebel zu besprühen. Die feinen Paclitaxelkristalle auf der Ballonoberfläche dienen als Kristallisationskeime für das neu aufgetragene Paclitaxel. Nach dem vierten Eintauchen des Ballons in die Paclitaxellösung lässt man das Methanol und DMSO verdunsten und trocknet danach den Ballon im Vakuum.

Falls erforderlich läßt sich der Restgehalt an Paclitaxel in der DMSO-Lösung bestimmen, so dass man über die bekannte Ausgangskonzentration die Menge an Paclitaxel bestimmen kann, welche auf dem Katheterballon aufgetragen worden ist.

In der Regel werden 1 µg bis 20 µg Paclitaxel pro mm² beschichteter Ballonoberfläche aufgetragen.

Nach Sterilisation wird der Ballon mit einer Schutzhülle versehen, welche den Wirkstoff auf dem beschichteten dilatierbaren Katheterballon während der Transport - und Lagerzeit schützen soll und vor dem Einführen des Katheters vom Kardiologen entfernt wird.

### Beispiel 3:

Ein kommerziell erhältlicher Dilatationskatheter mit expandierbarem Ballon aus einem Poly wird bereitgestellt.

Der Katheterballon besteht aus einem Polyamid-Polyether-Polyester-Blockcopolymer oder aus Polyurethan, einem Polyester oder einem Polyolefin.

Mittels eines Lasers werden Rinnen oder Vertiefungen oder Spiralen oder netzartige Muster mit einer Tiefe von 1 nm bis 1 µm in das Ballonmaterial gebrannt.

Eine Lösung von 70 mg Paclitaxel in 1,0 ml DMSO mit einem Wassergehalt von ca. 3 Vol.% wird angesetzt und mittels eines Streichverfahrens oder eines Spritzenverfahrens auf den horizontal liegenden Teil der Katheterballonoberfläche aufgetragen.

Nun wird entmineralisiertes Wasser, welches mit einer Mischung aus Weinsäure und Natriumtartrat auf einen pH-Wert von 4,8 eingestellt worden ist, mittels einer feinen Spritze unmittelbar auf die mit der Paclitaxellösung benetzten Oberfläche des Katheterballons aufgetragen.

In den Strukturierungen der Ballonoberfläche beginnt Paclitaxel sich abzusetzen. Nach der Verdunstung des Lösungsmittels DMSO wird der Katheterballon gedreht und ein weiterer nun horizontal liegender Teilbereich der Ballonoberfläche wird nun mit der Paclitaxel-DMSO-Lösung benetzt und danach wird wie vorher beschrieben, Wasser auf diese benetzte Oberfläche aufgebracht, bis das Paclitaxel ausfällt. Der Benetzungs- und Fällungsvorgang wird so oft wiederholt, bis die gesamte Ballonoberfläche beschichtet ist.

Danach wird der Katheterballon gut getrocknet und man erhält eine amorphe weitgehend in den Mikrostrukturierungen enthaltene Paclitaxel-Beschichtung.

Die Menge an Paclitaxel auf dem Katheterballon beträgt 2 bis 4 µg pro mm² Ballonoberfläche.

Nach Sterilisation wird der Ballon mit einer Schutzhülle versehen, welche den Wirkstoff auf dem beschichteten dilatierbaren Katheterballon während der Transport - und Lagerzeit schützen soll und vor dem Einführen des Katheters vom Kardiologen entfernt wird.

### Beispiel 4:

Auf den erfindungsgemäß beschichteten Katheterballon gemäß Beispiel 1 wird ein unbeschichteter Cobalt-Chrom-Stent aufgesetzt.

### Beispiel 5:

Auf den erfindungsgemäß beschichteten Katheterballon gemäß Beispiel 1 wird ein mit Paclitaxel beschichteter Cobalt-Chrom-Stent aufgesetzt.

### Beispiel 6:

Allgemeine Vorschrift zur Durchführung der Dilatation mit dem erfindungsgemäß beschichteten Katheterballon.

Der Katheterballon ist mit 3 µg festem Paclitaxel pro mm² Ballonoberfläche beschichtet. Ein durchschnittlicher Patient wird mit ca. 325 mg Acetylsalicylsäure und ca. 300 mg Clopidogrel 12 und 2 Stunden vor der Dilatation vorbehandelt. Vor dem Eingriff wird so viel Heparin intravenös verabreicht, dass die Koagulationszeit mehr als 250 Sekunden beträgt. Nun wurde eine vom Arzt zu bestimmende Menge eines Glykoprotein Ib/IIIa Inhibitors verabreicht und ein Führungsdraht wird bis zur Zielregion vorgeschoben. Danach wird der erfindungsgemäß beschichtete Katheterballon eingeführt und in der Zielregion mit 6 bis 8 atm (ca. 6000 bis 8000 hPa) für ca. 1 Minute dilatiert und sofern erforderlich deflatiert und weitere ein, zwei oder dreimal mit ca. 8 atm (8000 hPa) inflatiert.

### Beispiel 7:

Ein 66 Jahre alter Patient, der ehemaliger Raucher ist und an Hypercholerterinämie leidet, leidet ferner an Angina, welche nicht mit Atemnot und Schwindel einherging. Nach der Dokumentation von schweren Ischämien in peripheren Adern beim Phase III Bruce Streß Test wurde eine Angiographie Diagnose durchgeführt.

Die koronare Angiographie ergab eine Okklusion der RCA mit Kollateralisierung des linken Koronarsystems. Daraufhin wurde eine Rekanalisierung der RCA mit einem unbeschichteten Metallstent durchgeführt.

Nach ca. einem Jahr war der Patient noch immer asymptomatisch und es wurde ein Thallium-201 Belastungs-Stress Test durchgeführt, der eine Hypoperfusion in der unteren Gefäßwand zeigte.

Zwei Monate später wurde eine Koronarangiographie durchgeführt, welche eine diffuse In-Stent-Restenose (ISR) des proximalen, mittleren und distalen Abschnitts der RCA und eine Kollateralisierung der posterior abfallenden Koronararterie (PDA) mit einer 70%igen Stenose im mittleren Trakt.

Es wurde beim Patienten eine erneute PTCA mit einem Schlitzballon (cutting balloon) über die gesamte Länge der RCA gefolgt von einer Brachytherapie im proximalen, mittleren und distalen Bereicht der RCA und PDA Koronararterie durchgeführt unter Implantation eines unbeschichteten Metallstents in den mittleren Bereich der LCX.

Nach ca. 6 Monaten wurde eine Kontrollangiographie vorgenommen, welche der PTCA in den RCA und PDA Koronararterien eine gutes Zwischenergebnis bescheinigte jedoch verbunden mit einer Okklusion der PL Arterie, welche trotz mehrerer Versuche unter Verwendung verschiedenen Führungsdrähte nicht wieder geöffnet werden konnte.

Nach ca. drei Jahren unterzog sich der Patient erneut einem Stress Test und das EKG zeigte zwei Auffälligkeiten. Daraufhin wurde erneut eine Koronarangiographie vorgenommen, welche eine 70%ige ISR im proximalen Bereich und eine 95%ige ISR im distalen Bereich der RCA sowie eine 60%ige Stenose der PDA und PL Koronararterie zeigte.

Die proximalen und distalen Läsionen der RCA wurden mit einer Cypher Stent Implantation behandelt.

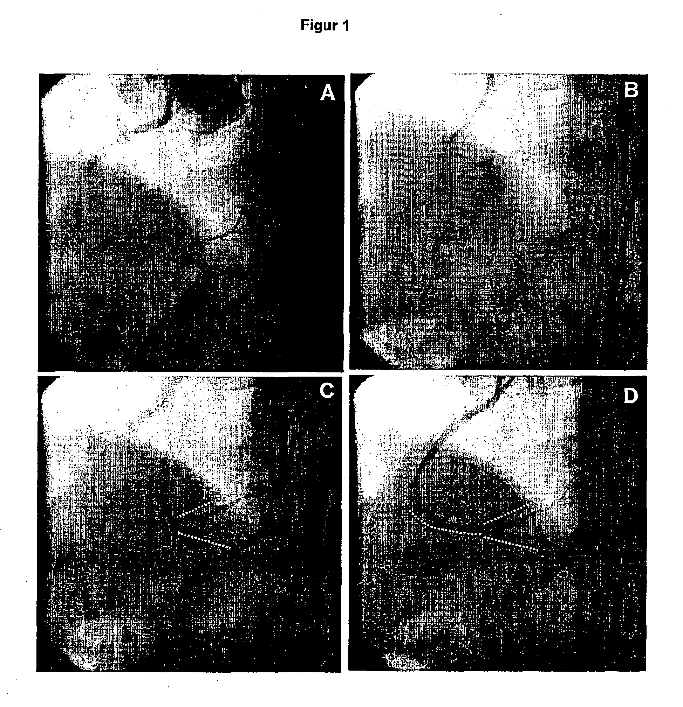

Nach weiteren ca. 2,5 Jahren wurde ein weiterer Stress Test durchgeführt, der wiederum Auffälligkeiten im inferior und postero-lateralen Bereich zeigte. Eine Koronarangiographie ergab diesmal eine vollständige Okklusion der RCA mit Kollateralisierung vom linken bis zum rechten Koronarsystem (Figur 1, Bild A) verbunden mit einer starken Atheromasie der PDA und PL Koronararterie.

Nach Einführung eines Judkins right 8Fr Führungskatheters und eines Judkins Diagnosekatheters wurde die ISR Okklusion mit einem Miracle 3 Führungsdraht durchstoßen, der bis in die PDA Koronararterie geführt wurde. Nach Vordilatation mit einem Avion 1.5x14 mm Ballon, wurden zwei Durchgänge mit einem direktionalen koronaren Atherektomie-Katheter 2,6 mm (FoxHollow, SilverHawks, California) erzeugt (Figur 1, Bild B), um einen Teil des neointimalen Gewebes zu entfernen. Daraufhin wurde ein Führungsdraht in die PL Koronararterie eingeführt.

Trotz der Anwesenheit mehrerer Stents gelang es einen 2,5x13 Paclitaxel eluierenden Katheterballon (DIOR, Eurocor, Germany) mittels der "kissing balloon"-Technik einzuführen und mit 14 atm (14186 hPa) für 2 Minuten zu dilatieren (Figur 1, Bild C). Nachfolgend wurden zwei Taxus 2,25x24 mm Stents gleichzeitig im proximalen Bereicht der PL und PDA Koronararterie und im distalen Bereich der RCA platziert und mit einem Druck von 22 atm (22292 hPa) wurde mit einem Paclitaxel eluierenden Katheterballon für 2 Minuten nachdilatiert. Danach wurde ein Taxus 3,5x32 mm Stent in den distalen Bereich der RCA und zwei Taxus 4,0x32 mm Stents in den mittleren und proximalen Bereich der RCA implantiert, welche ein sehr gutes sofortiges Angiographieergebnis erbrachten (Figur 1, Bild D). Innerhalb der nachfolgenden 12 Monate blieb der Patient asymptomatisch.

### Beispiel 8:

Ein 53 Jahre alter Patient, der Raucher ist, leidet an NIDDM und Dyslipidämie und ist die ischämische Herzerkrankungen in der Familie vorbelastet.

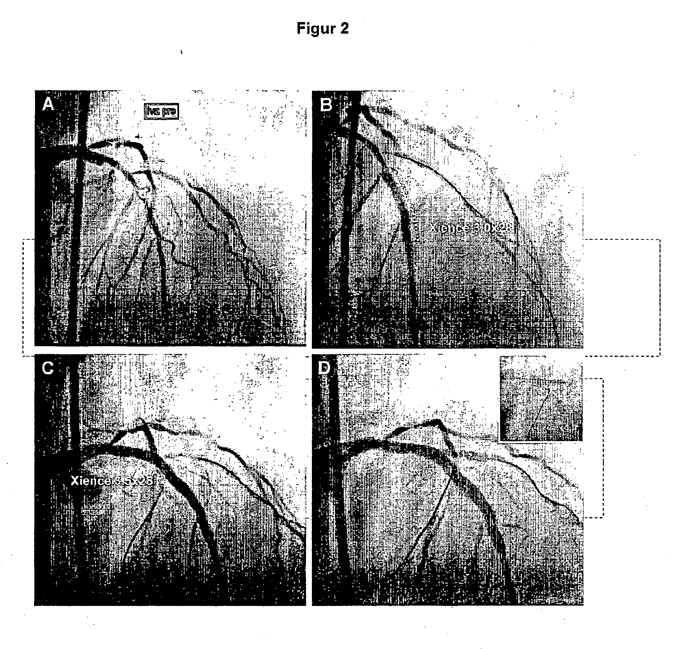

Der Patient erlitt 1993 einen akuten myokardialen Infarkt und wurde daraufhin einer primären PCI der LAD und LCX Koronararterie ohne Stentimplantation unterzogen. Der Patient war daraufhin ca. 14 Jahre asymptomatisch als er mit einer NSTEMI erneut ins Krankenhaus eingeliefert wurde. Die Koronarangiographie zeigte eine angiograpisch normale linke Hauptader und eine Bifurkationsläsion, welche die LAD/D1 einschließt, mit einer 90%igen Stenose der LAD, einer 80%igen Stenose der ersten Diagonalen (Figur 2, Bild A), ferner einer 90%igen Stenose der LCX im proximalen Bereich und einer 90%igen Stenose einer dominanten RCA im mittleren Bereich.

Nach einer Besprechung mit dem Patienten wurde eine vollständige Revaskularisierungsstrategie ausgewählt. Nachdem ein Führungsdraht in die LDA, die erste Diagonale und die zweite septale Verzweigung zum Schutz gelegt wurde, wurde eine IVUS durchgeführt, um die Bifurkationsläsion zu untersuchen und um die richtige Größe für Ballon und Stent auszuwählen. Die IVUS bestätigte das Ergebnis sowohl für die LDA als auch für die D1 und zeigte eine diffuse Erkrankung der LAD.

Um ein doppeltes Stentsetzen in der Bifurkation bei einem Diabetespatienten zu vermeiden, wurde eine provisorische Stenting-Technik für den Seitenarm gewählt. Ein Quantum 3,0x20 mm (Boston-Scientific) wurde für eine Prädilatation bei 20 atm (20265 hPa) gewählt, gefolgt von einer 3,0x28 mm Xience Stent (Abbott Vascular) Implantation (Figur 2, Bild B). Danach wurde der diagonale Seitenarm unter Verwendung eines DIOR Paclitaxel-eluierenden Ballon (Eurocor GmbH, Germany) 3,0x17 bei 10 atm (10133 hPa) für 2,0 Minuten dilatiert. Danach wurde ein 3,5x28 mm Xience Stent in die Bifurkationsläsion implantiert, gefolgt von einer abschließenden "kissing balloon"-Inflation (Quantum 3,5x13 der LAD bei 8 atm (8106 hPa) und DIOR Paclitaxel-eluierender Ballon 3,0x17 der D1 bei 10 atm (10133 hPa)). Es wurde ein exzellentes angiographisches Resultat erhalten (Figur 2, Bilder C und D).

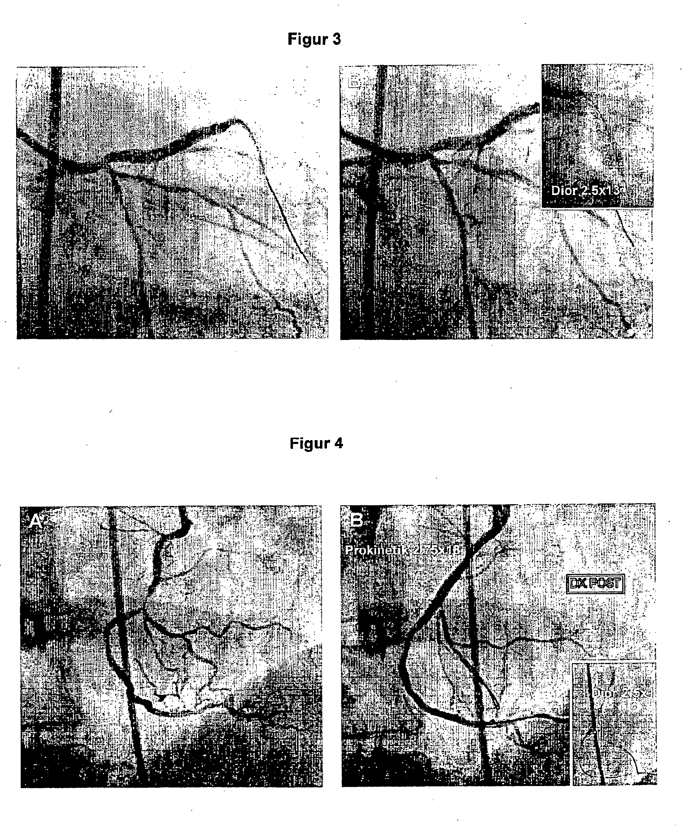

Nachfolgend wurde ein stentähnliches Ergebnis bei der Dilatation des proximalen Segments der LCX mit einer Multidilatationsstrategie unter Verwendung eines 2.5x13 mm DIOR Paclitaxel-eluierenden Ballons bei 16 atm (16212 hPa) und verlängerter Inflationen erzielt (totale Inflationszeit: 4,5 Minuten; Figur 3, Bilder A und B).

Abschließend wurde nach Schutz der akuten marginalen Verzeigung ein Führungsdraht in die Läsion der RCA Koronararterie und es wurde eine Prädilatation mit einem 2,5x13 mm DIOR Paclitaxel-eluierenden Ballon bei 14 atm (14186 hPa) für 1,45 Minuten vorgenommen. Danach wurde ein unbeschichteter Cobalt-Chrom-Stent mit dünnen Streben (Prokinetic 2,75x18 bei 8 atm (8106 hPa); Biotronic) implantiert und es ein sofortiges exzellentes Angiographieergebnis erhalten (Figur 4, Bilder A und B):

## Patentansprüche

1. Verfahren zur Beladung von dilatierbaren Katheterballons umfassend die folgenden Schritte:
I) Bereitstellung eines dilatierbaren Katheterballons,
II) Bereitstellung einer Lösung von Paclitaxel in Dimethylsulfoxid,
III) Durchführung einer Strukturierung der Oberfläche des dilatierbaren Katheterballons,
IV) Benetzung der Oberfläche des dilatierbaren Katheterballons mit der Lösung von Paclitaxel in Dimethylsulfoxid,
V) Zugabe von Wasser, wobei das Wasser einen pH-Wert von 3 bis 5 aufweist, welches in der Lage ist Paclitaxel auszufällen, auf die mit der Lösung von Paclitaxel in Dimethylsulfoxid benetzte Oberfläche des dilatierbaren Katheterballons,
VI) Trocknung der benetzten Oberfläche des dilatierbaren Katheterballons.

2. Verfahren nach Anspruch 1, wobei der dilatierbare Katheterballon im expandierten Zustand beschichtet wird.

3. Verfahren nach Anspruch 1, wobei es sich bei dem dilatierbaren Katheterballon um einen Faltenballon handelt, umfassend den Schritt VII):
VII) Rückfaltung des Faltenballons in den komprimierten Zustand.

4. Verfahren nach einem der vorherigen Ansprüche, wobei die Lösung von Paclitaxel in Dimethylsulfoxid 1 mg bis 150 mg Paclitaxel pro 1 ml Dimethylsulfoxid enthält.

5. Verfahren nach Anspruch 4, wobei die Lösung von Paclitaxel in Dimethylsulfoxid 40 mg bis 60 mg Paclitaxel pro 1 ml Dimethylsulfoxid enthält.

6. Verfahren nach einem der vorherigen Ansprüche, wobei das verwendete Dimethylsulfoxid einen Wassergehalt kleiner als 5 Vol.% aufweist.

7. Verfahren nach einem der vorherigen Ansprüche, wobei der Lösung aus Paclitaxel in Dimethylsulfoxid noch mindestens eine Trägersubstanz zugesetzt wird ausgewählt wird aus der Gruppe bestehend aus:
Parylen C, Parylen D, Parylen N, Parylen F, Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride, Polymaleinsäureanhydride, Polyhydroxymethacrylate, Fibrin, Polycyanoacrylate, Polycaprolactondimethylacrylate, Poly-β-Maleinsäure Polycaprolactonbutylacrylate, Multiblockpolymere aus Oligocaprolactondiole und Oligodioxanondiole, Polyetherestermultiblockpolymere aus PEG und Polybutylenterephtalat, Polypivotolactone, Polyglycolsäuretrimethylcarbonate Polycaprolactonglycolide, Poly(γ-ethylglutamat), Poly(DTH-Iminocarbonat), Poly(DTE-co-DT-carbonat), Poly(Bisphenol A-iminocarbonat), Polyorthoester, Polyglycolsäuretrimethylcarbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxidpropylenoxid, weiche Polyurethane, Polyurethane mit Aminosäurereste im Backbone, Polyetherester, Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, Polyhydroxyalkanoate, Pectinsäure, Actinsäure, Carboxymethylsulfat, Albumin, Hyaluronsäure, Chitosan und seine Derivate, Heparansulfate und seine Derivate, Heparine, Chondroitinsulfat, Dextran, ß-Cyclodextrine, Copolymere mit PEG und Polypropylenglycol, Gummi arabicum, Guar, Gelatine, Collagen Collagen-N-Hydroxysuccinimid, Lipide, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane, Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone, Polysiloxane, Polydimethylsiloxane, Polyvinylhalogene und Copolymere, Celluloseether, Cellulosetriacetate, Chitosane und Copolymere und/oder Mischungen der vorgenannten Polymere.

8. Verfahren nach einem der vorherigen Ansprüche, wobei die Schritte IV) bis VI) mehrmals wiederholt werden.

9. Verfahren nach einem der vorherigen Ansprüche, wobei der dilatierbare Katheterballon aus einem Material oder einer Materialmischung besteht, wobei das Material oder die Materialmischung aus der folgenden Gruppe an Materialien ausgewählt wird:
Parylen C, Parylen D, Parylen N, Parylen F, Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride, Polymaleinsäureanhydride, Polyhydroxymethacrylate, Fibrin, Polycyanoacrylate, Polycaprolactondimethylacrylate, Poly-β-Maleinsäure Polycaprolactonbutylacrylate, Multiblockpolymere aus Oligocaprolactondiole und Oligodioxanondiole, Polyetherestermultiblockpolymere aus PEG und Polybutylenterephtalat, Polypivotolactone, Polyglycolsäuretrimethylcarbonate Polycaprolactonglycolide, Poly(γ-ethylglutamat), Poly(DTH-Iminocarbonat), Poly(DTE-co-DT-carbonat), Poly(Bisphenol A-iminocarbonat), Polyorthoester, Polyglycolsäuretrimethylcarbonate, Polytrimethylcarbonate Polyiminocarbonate, Poly(N-vinyl)-Pyrrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxidpropylenoxid, weiche Polyurethane, Polyurethane mit Aminosäurereste im Backbone, Polyetherester, Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, Polyhydroxyalkanoate, Pectinsäure, Actinsäure, Carboxymethylsulfat, Albumin, Hyaluronsäure, Chitosan und seine Derivate, Heparansulfate und seine Derivate, Heparine, Chondroitinsulfat, Dextran, ß-Cyclodextrine, Copolymere mit PEG und Polypropylenglycol, Gummi arabicum, Guar, Gelatine, Collagen Collagen-N-Hydroxysuccinimid, Lipide, Phospholipide, Polyacrylsäure, Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane, Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone, Polysiloxane, Polydimethylsiloxane, Polyvinylhalogene und Copolymere, Celluloseether, Cellulosetriacetate, Chitosane und Copolymere und/oder Mischungen der vorgenannten Polymere.

10. Verfahren nach einem der vorherigen Ansprüche, wobei in der Lösung von Paclitaxel in Dimethylsulfoxid noch ein weiterer Wirkstoff aufgenommen wird, ausgewählt aus der Gruppe bestehend aus:
Abciximab, Acemetacin, Acetylvismion B, Aclarubicin, Ademetionin, Adriamycin, Aescin, Afromoson, Akagerin, Aldesleukin, Amidoron, Aminoglutethemid, Amsacrin, Anakinra, Anastrozol, Anemonin, Anopterin, Antimykotika, Antithrombotika, Apocymarin, Argatroban, Aristolactam-All, Aristolochsäure, Ascomycin, Asparaginase, Aspirin, Atorvastatin, Auranofin, Azathioprin, Azithromycin, Baccatin, Bafilomycin, Basiliximab, Bendamustin, Benzocain, Berberin, Betulin, Betulinsäure, Bilobol, Bisparthenolidin, Bleomycin, Bombrestatin, Boswellinsäuren und ihre Derivate, Bruceanole A,B und C, Bryophyllin A, Busulfan, Antithrombin, Bivalirudin, Cadherine, Camptothecin, Capecitabin, o-Carbamoylphenoxyessigsäure, Carboplatin, Carmustin, Celecoxib, Cepharantin, Cerivastatin, CETP-Inhibitoren, Chlorambucil, Chloroquinphosphat, Cictoxin, Ciprofloxacin, Cisplatin, Cladribin, Clarithromycin, Colchicin, Concanamycin, Coumadin, C-Type Natriuretic Peptide (CNP), Cudraisoflavon A, Curcumin, Cyclophosphamid, Cyclosporin A, Cytarabin, Dacarbazin, Daclizumab, Dactinomycin, Dapson, Daunorubicin, Diclofenac, 1,11-Dimethoxycanthin-6-on, Docetaxel, Doxorubicin, Dunaimycin, Epirubicin, Epothilone A und B, Erythromycin, Estramustin, Etobosid, Everolimus, Filgrastim, Fluroblastin, Fluvastatin, Fludarabin, Fludarabin-5'-dihydrogenphosphat, Fluorouracil, Folimycin, Fosfestrol, Gemcitabin, Ghalakinosid, Ginkgol, Ginkgolsäure, Glykosid 1a, 4-Hydroxyoxycyclophosphamid, Idarubicin, Ifosfamid, Josamycin, Lapachol, Lomustin, Lovastatin, Melphalan, Midecamycin, Mitoxantron, Nimustin, Pitavastatin, Pravastatin, Procarbazin, Mitomycin, Methotrexat, Mercaptopurin, Thioguanin, Oxaliplatin, Irinotecan, Topotecan, Hydroxycarbamid, Miltefosin, Pentostatin, Pegasparase, Exemestan, Letrozol, Formestan, Mitoxanthrone, Mycophenolatmofetil, ß-Lapachon, Podophyllotoxin, Podophyllsäure-2-ethylhydrazid, Molgramostim (rhuGM-CSF), Peginterferon α-2b, Lanograstim (r-HuG-CSF), Macrogol, Selectin (Cytokinantagonist), Cytokininhibitoren, COX-2-Inhibitor, Angiopeptin, monoklonale Antikörper, die die Muskelzellproliferation hemmen, bFGF-Antagonisten, Probucol, Prostaglandine, 1-Hydroxy-11-Methoxycanthin-6-on, Scopolectin, NO-Donoren, Pentaerythrityltetranitrat und Syndnoeimine, S-Nitrosoderivate, Tamoxifen, Staurosporin, ß-Estradiol, α-Estradiol, Estriol, Estron, Ethinylestradiol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Paclitaxel und dessen Derivate, 6-α-Hydroxy-Paclitaxel, Taxotere, Mofebutazon, Lonazolac, Lidocain, Ketoprofen, Mefenaminsäure, Piroxicam, Meloxicam, Penicillamin, Hydroxychloroquin, Natriumaurothiomalat, Oxaceprol, β-sitosterin, Myrtecain, Polidocanol, Nonivamid, Levomenthol, Ellipticin, D-24851 (Calbiochem), Colcemid, Cytochalasin A-E, Indanocine, Nocadazole, Bacitracin, Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika, Cefadroxil, Cefazolin, Cefaclor, Cefotixin Tobramycin, Gentamycin, Penicilline, Dicloxacillin, Oxacillin, Sulfonamide, Metronidazol, Enoxoparin, Heparin, Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin, Seramin, ACE-Inhibitoren, Captopril, Cilazapril, Lisinopril, Enalapril, Losartan, Thioproteaseinhibitoren, Prostacyclin, Vapiprost, Interferon α, β und γ, Histaminantagonisten, Serotoninblocker, Apoptoseinhibitoren, Apoptoseregulatoren, Halofuginon, Nifedipin, Tocopherol Tranilast, Molsidomin, Teepolyphenole, Epicatechingallat, Epigallocatechingallat, Leflunomid, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, natürliche und synthetisch hergestellte Steroide, Inotodiol, Maquirosid A, Ghalakinosid, Mansonin, Streblosid, Hydrocortison, Betamethason, Dexamethason, nichtsteroidale Substanzen (NSAIDS), Fenoporfen, Ibuprofen, Indomethacin, Naproxen, Phenylbutazon, antivirale Agentien, Acyclovir, Ganciclovir, Zidovudin, Clotrimazol, Flucytosin, Griseofulvin, Ketoconazol, Miconazol, Nystatin, Terbinafin, antiprozoale Agentien, Chloroquin, Mefloquin, Quinin, natürliche Terpenoide, Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceantinoside C, Yadanzioside N, und P, Isodeoxyelephantopin, Tomenphantopin A und B, Coronarin A,B,C und D, Ursolsäure, Hyptatsäure A, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Hydroxyanopterin, Protoanemonin, Cheliburinchlorid, Sinococulin A und B, Dihydronitidin, Nitidinchlorid, 12-beta-Hydroxypregnadien 3,20-dion, Helenalin, Indicin, Indicin-N-oxid, Lasiocarpin, Inotodiol, , Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Periplocosid A, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Dihydrousambaraensin, Hydroxyusambarin, Strychnopentamin, Strychnophyllin, Usambarin, Usambarensin, Liriodenin, Oxoushinsunin, Daphnoretin, Lariciresinol, Methoxylariciresinol, Syringaresinol, Sirolimus (Rapamycin), Somatostatin, Tacrolimus, Roxithromycin, Troleandomycin, Simvastatin, Rosuvastatin, Vinblastin, Vincristin, Vindesin, Teniposid, Vinorelbin, Tropfosfamid, Treosulfan, Tremozolomid, Thiotepa, Tretinoin, Spiramycin, Umbelliferon, Desacetylvismion A, Vismion A und B, Zeorin.

## Claims

1. Method for loading dilatable catheter balloons comprising the following steps:
I) providing a dilatable catheter balloon;
II) providing a solution of paclitaxel in dimethyl sulfoxide;
III) texturing the surface of the dilatable catheter balloon;
IV) wetting the surface of the dilatable catheter balloon with the solution of paclitaxel in dimethyl sulfoxide;
V) applying water, wherein the water has a pH of 3 to 5, which is capable of precipitating paclitaxel onto the surface of the dilatable catheter balloon wetted with the solution of paclitaxel in dimethyl sulfoxide;
VI) drying the wetted surface of the dilatable catheter balloon.

2. Method according to claim 1, wherein the dilatable catheter balloon is coated in its expanded state.

3. Method according to claim 1, wherein the dilatable catheter balloon is a multifold balloon comprising step VII):
VII) refolding of the multifold balloon into its compressed state.

4. Method according to any one of the preceding claims, wherein the solution of paclitaxel in dimethyl sulfoxide contains from 1 mg to 150 mg of paclitaxel per 1 ml of dimethyl sulfoxide.

5. Method according to claim 4, wherein the solution of paclitaxel in dimethyl sulfoxide contains from 40 mg to 60 mg of paclitaxel per 1 ml of dimethyl sulfoxide.

6. Method according to any one of the preceding claims, wherein the dimethyl sulfoxide used has a water content of less than 5 percent by volume.

7. Method according to any one of the preceding claims, wherein at least one carrier substance is added to the solution of paclitaxel in dimethyl sulfoxide selected from the group consisting of:
parylene C, parylene D, parylene N, parylene F, polyvalerolactones, poly-ε-decalactone, polylactonic acid, polyglycolic acid, polylactides, polyglycolides, copolymers of the polylactides and polyglycolides, poly-ε-caprolactone, polyhydroxybutyric acid, polyhydroxybutyrates, polyhydroxyvalerates, polyhydroxybutyrate-co-valerate, poly(1,4-dioxane-2,3-dione), poly(1,3-dioxane-2-one), poly-para-dioxanone, polyanhydrides, polymaleic acid anhydride, polyhydroxymethacrylates, fibrin, polycyanoacrylate, polycaprolactone dimethylacrylates, poly-ß-maleic acid, polycaprolactone butyl acrylates, multiblock polymers from oligocaprolactonedioles and oligodioxanonedioles, polyether ester multiblock polymers from PEG and poly(butylene terephthalate), polypivotolactones, polyglycolic acid trimethyl carbonates, polycaprolactone glycolides, poly(γ-ethyl glutamate), poly(DTH-iminocarbonate), poly(DTE-co-DT-carbonate), poly(bisphenol A-iminocarbonate), polyorthoesters, polyglycolic acid trimethyl-carbonate, polytrimethyl carbonates, polyiminocarbonates, poly(N-vinyl)-pyrrolidone, polyvinyl alcohols, polyester amides, glycolized polyesters, polyphosphoesters, polyphosphazenes, poly[p-carboxyphenoxy)propane], polyhydroxy pentanoic acid, polyanhydrides, polyethylene oxide propylene oxide soft polyurethanes, polyurethanes having amino acid residues in the backbone, polyether esters, polyethylene oxide, polyalkene oxalates, polyorthoesters as well as their copolymers, lipids, carrageenans, fibrinogen, starch, collagen, protein based polymers, polyamino acids, synthetic polyamino acids, zein, polyhydroxyalkanoates, pectic acid, actinic acid, carboxymethyl sulfate, albumin, hyaluronic acid, chitosan and derivatives thereof, heparan sulfates and derivatives thereof, heparins, chondroitin sulfate, dextran, ß-cyclodextrins, copolymers with PEG and polypropylene glycol, gum arabic, guar, gelatin, collagen collagen N-hydroxysuccinimide, lipids, phospholipids, polyacrylic acid, polyacrylates, polymethyl methacrylate, polybutyl methacrylate, polyacrylamide, polyacrylonitriles, polyamides, polyetheramides, polyethylene amine, polyimides, polycarbonates, polycarbourethanes, polyvinyl ketones, polyvinyl halogenides, polyvinylidene halogenides, polyvinyl ethers, polyisobutylenes, polyvinyl aromatics, polyvinyl esters, polyvinyl pyrrolidones, polyoxymethylene, polytetramethylene oxide, polyethylene, polypropylene, polytetrafluoroethylene, polyurethanes, polyether urethanes, silicone polyether urethanes, silicone polyurethanes, silicone polycarbonate urethanes, polyolefin elastomers, polyisobutylenes, EPDM gums, fluorosilicones, carboxymethyl chitosans, polyaryletheretherketones, polyetheretherketones, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, EPDM gums, silicones, polysiloxanes, polydimethylsiloxanes, polyvinyl halogens and copolymers, cellulose ethers, cellulose triacetates, chitosans and copolymers and/ or mixtures of the aforementioned polymers.

8. Method according to any one of the preceding claims, wherein steps IV) to VI) are repeated several times.

9. Method according to any one of the preceding claims, wherein the dilatable catheter balloon consists of a material or a mixture of materials, wherein the material or the mixture of materials is selected from the following group of materials:
parylene C, parylene D, parylene N, parylene F, polyvalerolactones, poly-ε-decalactone, polylactonic acid, polyglycolic acid, polylactides, polyglycolides, copolymers of the polylactides and polyglycolides, poly-ε-caprolactone, polyhydroxybutyric acid, polyhydroxybutyrates, polyhydroxyvalerates, polyhydroxybutyrate-co-valerate, poly(1,4-dioxane-2,3-dione), poly(1,3-dioxane-2-one), poly-para-dioxanone, polyanhydrides, polymaleic acid anhydride, polyhydroxymethacrylates, fibrin, polycyanoacrylate, polycaprolactone dimethylacrylates, poly-ß-maleic acid, polycaprolactone butyl acrylates, multiblock polymers from oligocaprolactonedioles and oligodioxanonedioles, polyether ester multiblock polymers from PEG and poly(butylene terephthalate), polypivotolactones, polyglycolic acid trimethyl carbonates, polycaprolactone glycolides, poly(γ-ethyl glutamate), poly(DTH-iminocarbonate), poly(DTE-co-DT-carbonate), poly(bisphenol A-iminocarbonate), polyorthoesters, polyglycolic acid trimethyl-carbonate, polytrimethyl carbonates, polyiminocarbonates, poly(N-vinyl)-pyrrolidone, polyvinyl alcohols, polyester amides, glycolized polyesters, polyphosphoesters, polyphosphazenes, poly[p-carboxyphenoxy)propane], polyhydroxy pentanoic acid, polyanhydrides, polyethylene oxide propylene oxide, soft polyurethanes, polyurethanes having amino acid residues in the backbone, polyether ester, polyethylene oxide, polyalkene oxalates, polyorthoesters as well as their copolymers, lipids, carrageenans, fibrinogen, starch, collagen, protein based polymers, polyamino acids, synthetic polyamino acids, zein, polyhydroxyalkanoates, pectic acid, actinic acid, carboxymethyl sulfate, albumin, hyaluronic acid, chitosan and derivatives thereof, heparan sulfates and derivatives thereof, heparins, chondroitin sulfate, dextran, ß-cyclodextrins, copolymers with PEG and polypropylene glycol, gum arabic, guar, gelatine, collagen collagen N-hydroxysuccinimide, lipids, phospholipids, polyacrylic acid, polyacrylates, polymethyl methacrylate, polybutyl methacrylate, polyacrylamide, polyacrylonitriles, polyamides, polyetheramides, polyethylene amine, polyimides, polycarbonates, polycarbourethanes, polyvinyl ketones, polyvinyl halogenides, polyvinylidene halogenides, polyvinyl ethers, polyisobutylenes, polyvinyl aromatics, polyvinyl esters, polyvinyl pyrrolidones, polyoxymethylene, polytetramethylene oxide, polyethylene, polypropylene, polytetrafluoroethylene, polyurethanes, polyether urethanes, silicone polyether urethanes, silicone polyurethanes, silicone polycarbonate urethanes, polyolefin elastomers, polyisobutylenes, EPDM gums, fluorosilicones, carboxymethyl chitosans, polyaryletheretherketones, polyetheretherketones, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, EPDM gums, silicones, polysiloxanes, polydimethylsiloxanes, polyvinyl halogens and copolymers, cellulose ethers, cellulose triacetates, chitosans and copolymers and/ or mixtures of the aforementioned polymers.

10. Method according to any one of the preceding claims, wherein an additional active agent is incorporated into the solution of paclitaxel in dimethyl sulfoxide, selected from the group consisting of:
abciximab, acemetacin, acetylvismione B, aclarubicin, ademetionine, adriamycin, aescin, afromosone, akagerine, aldesleukin, amidorone, aminoglutethimide, amsacrine, anakinra, anastrozole, anemonin, anopterine, antimycotics antithrombotics, apocymarin, argatroban, aristolactam-All, aristolochic acid, ascomycin, asparaginase, aspirin, atorvastatin, auranofin, azathioprine, azithromycin, baccatin, bafilomycin, basiliximab, bendamustine, benzocaine, berberine, betulin, betulinic acid, bilobol, bisparthenolidine, bleomycin, bombrestatin, Boswellic acids and derivatives thereof, bruceanol A, B and C, bryophyllin A, busulfan, antithrombin, bivalirudin, cadherins, camptothecin, capecitabine, o-carbamoyl-phenoxyacetic acid, carboplatin, carmustine, celecoxib, cepharanthin, cerivastatin, CETP inhibitors, chlorambucil, chloroquine phosphate, cicutoxin, ciprofloxacin, cisplatin, cladribine, clarithromycin, colchicine, concanamycin, coumadin, C-type natriuretic peptide (CNP), cudraisoflavone A, curcumin, cyclophosphamide, ciclosporin A, cytarabine, dacarbazine, daclizumab, dactinomycin, dapsone, daunorubicin, diclofenac, 1,11-dimethoxycanthin-6-one, docetaxel, doxorubicin, daunamycin, epirubicin, epothilone A and B, erythromycin, estramustine, etoposide, everolimus, filgrastim, fluroblastin, fluvastatin, fludarabine, fludarabine-5'-dihydrogen phosphate, fluorouracil, folimycin, fosfestrol, gemcitabine, ghalakinoside, ginkgol, ginkgolic acid, glycoside 1a, 4-hydroxyoxycyclo phosphamide, idarubicin, ifosfamide, josamycin, lapachol, lomustine, lovastatin, melphalan, midecamycin, mitoxantrone, nimustine, pitavastatin, pravastatin, procarbazine, mitomycin, methotrexate, mercaptopurine, thioguanine, oxaliplatin, irinotecan, topotecan, hydroxycarbamide, miltefosine, pentostatin, pegaspargase, exemestane, letrozole, formestane, mitoyanthrone, mycophenolate mofetil, β-lapachone, podophyllotoxin, podophyllic acid-2-ethyl hydrazide, molgramostim (rhuGM-CSF), peginterferon α-2b, lenograstim (r-HuG-CSF), macrogol, selectin (cytokine antagonist), cytokinin inhibitors, COX-2 inhibitor, angiopeptin, monoclonal antibodies inhibiting muscle cell proliferation, bFGF antagonists, probucol, prostaglandins, 1-hydroxy-11-methoxycanthin-6-one, scopoletin, NO donors, pentaerythrityl tetranitrate and sydnoimines, S-nitroso derivatives, tamoxifen, staurosporine, β-estradiol, α-estradiol, estriol, estrone, ethinyl estradiol, medroxyprogesterone, estradiol cypionates, estradiol benzoates, tranilast, kamebakaurin and other terpenoids used in cancer therapy, verapamil, tyrosine kinase inhibitors (tyrphostins), paclitaxel and derivatives thereof, 6-α-hydroxy-paclitaxel, taxoteres, mofebutazone, lonazolac, lidocaine, ketoprofen, mefenamic acid, piroxicam, meloxicam, penicillamine, hydroxychloroquine, sodium aurothiomalate, oxaceprol, β-sitosterol, myrtecaine, polidocanol, nonivamide, levomenthol, ellipticine, D-24851 (Calbiochem), colcemid, cytochalasin A-E, indanocine, nocodazole, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plasminogen activator inhibitor 1, plasminogen activator inhibitor 2, antisense oligonucleotides, VEGF inhibitors, IGF-1, active agents from the group of antibiotics, cefadroxil, cefazolin, cefaclor, cefoxitin, tobramycin, gentamicin, penicillins, dicloxacillin, oxacillin, sulfonamides, metronidazole, enoxaparin, heparin, hirudin, PPACK, protamine, prourokinase, streptokinase, warfarin, urokinase, vasodilators, dipyramidole, trapidil, nitroprussides, PDGF antagonists, triazolopyrimidine, seramin, ACE inhibitors, captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators, halofuginone, nifedipine, tocopherol, tranilast, molsidomine, tea polyphenols, epicatechin gallate, epigallocatechin gallate, leflunomide, etanercept, sulfasalazine, etoposide, dicloxacillin, tetracycline, triamcinolone, mutamycin, procainimide, retinoic acid, quinidine, disopyrimide, flecainide, propafenone, sotalol, natural and synthetically obtained steroids, inotodiol, maquiroside A, ghalakinoside, mansonine, strebloside, hydrocortisone, betamethasone, dexamethasone, non-steroidal substances (NSAIDS), fenoprofen, ibuprofen, indomethacin, naproxen, phenylbutazone, antiviral agents, acyclovir, ganciclovir, zidovudine, clotrimazole, flucytosine, griseofulvin, ketoconazole, miconazole, nystatin, terbinafine, antiprotozoal agents, chloroquine, mefloquine, quinine, natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid baccharinoids B1, B2, B3 and B7, tubeimoside, bruceantinoside C, yadanziosides N and P, isodeoxyelephantopin, tomenphantopin A and B, coronarin A,B C and D, ursolic acid, hyptatic acid A, iso-iridogermanal, maytenfoliol, effusantin A, excisanin A and B, longikaurin B, sculponeatin C, kamebaunin, leukamenin A and B, 13,18-dehydro-6-alpha-senecioyloxychaparrin, taxamairin A and B, regenilol, triptolide, cymarin, hydroxyanopterine, protoanemonin, cheliburin chloride, sinococuline A and B, dihydronitidine, nitidine chloride, 12-β-hydroxypregnadien-3,20-dione, helenalin, indicine, indicine-N-oxide, lasiocarpine, inotodiol, podophyllotoxin, justicidin A and B, larreatin, malloterin, mallotochromanol, isobutyrylmallotochromanol, marchantin A, maytansin, lycoridicin, margetine, pancratistatin, liriodenine, oxoushinsunine, periplocoside A, ursolic acid, deoxypsorospermin, psychorubin, ricin A, sanguinarine, manwu wheat acid, methylsorbifolin, chromones of spathelia, stizophyllin, mansonin, strebloside dihydrousambaraensine, hydroxyusambarine, strychnopentamine, strychnophylline, usambarine, usambarensine, liriodenine, daphnoretin, lariciresinol, methoxylariciresinol, syringaresinol, sirolimus (rapamycin), somatostatin, tacrolimus, roxithromycin, troleandomycin, simvastatin, rosuvastatin, vinblastine, vincristine, vindesine, teniposide, vinorelbine, trofosfamide, treosulfan, temozolomide, thiotepa, tretinoin, spiramycin, umbelliferone, desacetylvismione A, vismione A and B, zeorin.

## Revendications

1. Méthode de revêtement d'un ballonnet de cathéter dilatable comprenant les étapes suivantes:
I) provisionner un ballonnet de cathéter dilatable,
II) provisionner une solution de paclitaxel dans du diméthylsulfoxyde
III) exécution d'une structuration de la surface du ballonnet de cathéter dilatable,
IV) mouiller la surface du ballonnet de cathéter dilatable avec la solution de paclitaxel dans le diméthylsulfoxyde,
V) addition d'eau, où l'eau a un pH de 3 à 5, qui est capable précipité le paclitaxel, dans ce surface du ballonnet de cathéter dilatable, qui est humidifié avec la solution de paclitaxel dans le diméthylsulfoxyde,
VI) séchant la surface du ballonnet de cathéter dilatable.

2. Méthode selon la revendication 1, où le ballonnet de cathéter dilatable est revêtu dans l'état expansé.

3. Méthode selon la revendication 1, où le ballonnet de cathéter dilatable pliées multiples comprenant l'étape suivante:
VII) repliement des ballons pliées multiples dans l'état comprimé.

4. Méthode selon une revendication précédentes, où la solution de paclitaxel dans le diméthylsulfoxyde compris 1 mg à 150 mg paclitaxel par ml de diméthylsulfoxyde.

5. Méthode selon la revendication 4, où la solution de paclitaxel dans le diméthylsulfoxyde compris 40 mg à 60 mg paclitaxel par ml de diméthylsulfoxyde.

6. Méthode selon une revendication précédentes, où le diméthylsulfoxyde utilisé a une teneur en eau de moins de 5 pour cent volumiques.

7. Méthode selon une revendication précédentes, où au moins une substance de support est ajouté dans la solution de paclitaxel dans le diméthylsulfoxyde et cette substance est choisi parmi le groupe comprenant les substances suivantes:
parylène C, parylène D, parylène N, parylène F, polyvalérolactones, poly-ε-décalactones, polyacide lactonique, acide polyglycolique, polylactides, polyglycolides, copolymères des polylactides et polyglycolides, poly-ε-caprolactone, polyhydroxy acide butyrique, polyhydroxybutyrates, polyhydroxyvalérates, polyhydroxybutyrates-co-valérates, poly(1,4-dioxane-2,3-dione), poly(1,3-dioxane-2-dione), poly-para-dioxanone, polyanhydrides, anhydrides de l'acide polymaléique, polyhydroxyméthacrylates, fibrine, polycyanoacrylates, polycaprolactone diméthylacrylates, poly-β-acide maléique, polycaprolactone butyl acrylates, polymères séquences multiples d'oligocaprolactone diols et oligodioxanone diols, polymères séquencés multiples de polyéthers PEG et poly(butylène téréphtalate), polypivotolactones, triméthyl carbonates de l'acide polyglycolique, polycaprolactone-glycolides, poly(γ-éthylglutamate), poly(DTH-imino-carbonate), poly(DTE-co-DT-carbonate), poly(bisphenol A-imino-carbonate), polyorthoesters, poly(triméthyl-carbonates), poly(imino-carbonates), poly(N-vinyle)-pyrrolidone, alcools polyvinyliques, polyesteramides, polyesters glycolés, poly(phosphoesters), polyphosphazènes, poly[p-carboxyphénoxy)propane], polyhydroxy-acide valérique, polyanhydrides, oxyde de polyéthylène-oxyde de propylène, polyuréthanes souples, polyuréthanes ayant des résidus d'acide aminé dans la chaîne principale, polyétheresters, oxyde de polyéthylène, poly(alcène oxalate) polyorthoesters ainsi que leurs copolymères, lipides, carraghénanes, fibrinogène, amidon, collagène, polymères à base de protéines, acides polyaminés, acides polyaminés synthétiques, zéine, polyhydroxyalcanoates, acide pectique, acide actique, sulfate de carboxyméthyl, albumine, acide hyaluronique, chitosane et ses dérivés, sulfates d'héparane et leurs dérivés, héparines, sulfate de chondroïtine, dextrane, β-cyclodextrines et copolymères avec PEG et polypropylène glycol, gomme arabique, guar, gelatine, collagène collagène-N-hydroxy succinimide, lipides, phospholipides, acide polyacrylique, polyacrylates, polyméthylméthacrylate, polybutylméthacrylate, polyacrylamide, polyacrylonitriles, polyamides, polyétheramides, polyéthylène amine, polyimides, polycarbonates, polycarbouréthane, cétones de polyvinyle, halogénides de polyvinyle, halogénides de polyvinylidène, éthers de polyvinyle, polyisobutylènes, aromatiques polyvinyliques, esters polyvinyliques, pyrrolidones de polyvinyle, polyoxymethylène, oxyde de polytétraméthylène, polyéthylène, polypropylène, polytétrafluoroéthylène, polyuréthanes, polyétheruréthanes, polyétheruréthanes de silicone, polyuréthanes de silicone, polycarbonate-uréthanes de silicone, élastomères de polyoléfine, polyisobutylène, gommes EPDM, fluorosilicones, chitosanes de carboxyméthyl, poly(aryléther éther cétones), poly(éther éther cétones), poly(éthylène téréphtalate), polyvalérates, cellulose de carboxyméthyl, cellulose, viscose, triacétates de viscose, nitrates de cellulose, acétates de cellulose, cellulose de hydroxyéthyle, butyrates de cellulose, acetate-butyrates de cellulose, copolymères d'éthyle-vinyle acétate, polysulfones, résines époxy, résines ABS, gommes EPDM, silicones, polysiloxanes, polydiméthyl siloxanes, halogènes de polyvinyle et copolymères, éthers de cellulose, triacétates de cellulose, chitosane et copolymères et/ ou mélanges de ceux-ci.

8. Méthode selon une revendication précédentes, où les étapes IV) à VI) sont répétés plusieurs fois.

9. Méthode selon une revendication précédentes, où le ballonnet de cathéter dilatable consiste d'un matériau ou un mélange de matières, où le matériau ou le mélange de matières est choisi parmi le groupe suivant de matières:
parylène C, parylène D, parylène N, parylène F, polyvalérolactones, poly-ε-décalactones, polyacide lactonique, acide polyglycolique, polylactides, polyglycolides, copolymères des polylactides et polyglycolides, poly-ε-caprolactone, polyhydroxy acide butyrique, polyhydroxybutyrates, polyhydroxyvalérates, polyhydroxybutyrates-co-valérates, poly(1,4-dioxane-2,3-dione), poly(1,3-dioxane-2-dione), poly-para-dioxanone, polyanhydrides, anhydrides de l'acide polymaléique, polyhydroxyméthacrylates, fibrine, polycyanoacrylates, polycaprolactone diméthylacrylates, poly-β-acide maléique, polycaprolactone butyl acrylates, polymères séquencés multiples d'oligocaprolactone diols et oligodioxanone diols, polymères séquencés multiples de polyéthers PEG et poly(butylène téréphtalate), polypivotolactones, triméthyl carbonates de l'acide polyglycolique, polycaprolactone-glycolides, poly(γ-éthylglutamate), poly(DTH-imino-carbonate), poly(DTE-co-DT-carbonate), poly(bisphenol A-imino-carbonate), polyorthoesters, poly(triméthyl-carbonates), poly(imino-carbonates), poly(N-vinyle)-pyrrolidone, alcools polyvinyliques, polyesteramides, polyesters glycolés, poly(phosphoesters), polyphosphazènes, poly[p-carboxyphénoxy)propane], polyhydroxy-acide valérique, polyanhydrides, oxyde de polyéthylène-oxyde de propylène, polyuréthanes souples, polyuréthanes ayant des résidus d'acide aminé dans la chaîne principale, polyétheresters, oxyde de polyéthylène, poly(alcène oxalate) polyorthoesters ainsi que leurs copolymères, lipides, carraghénanes, fibrinogène, amidon, collagène, polymères à base de protéines, acides polyaminés, acides polyaminés synthétiques, zéine, polyhydroxyalcanoates, acide pectique, acide actique, sulfate de carboxyméthyl, albumine, acide hyaluronique, chitosane et ses dérivés, sulfates d'héparane et leurs dérivés, héparines, sulfate de chondroïtine, dextrane, β-cyclodextrines et copolymères avec PEG et polypropylène glycol, gomme arabique, guar, gelatine, collagène collagène-N-hydroxy succinimide, lipides, phospholipides, acide polyacrylique, polyacrylates, polyméthylméthacrylate, polybutylméthacrylate, polyacrylamide, polyacrylonitriles, polyamides, polyétheramides, polyéthylène amine, polyimides, polycarbonates, polycarbouréthane, cétones de polyvinyle, halogénides de polyvinyle, halogénides de polyvinylidène, éthers de polyvinyle, polyisobutylènes, aromatiques polyvinyliques, esters polyvinyliques, pyrrolidones de polyvinyle, polyoxymethylène, oxyde de polytétraméthylène, polyéthylène, polypropylène, polytétrafluoroéthylène, polyuréthanes, polyétheruréthanes, polyétheruréthanes de silicone, polyuréthanes de silicone, polycarbonate-uréthanes de silicone, élastomères de polyoléfine, polyisobutylène, gommes EPDM, fluorosilicones, chitosanes de carboxyméthyl, poly(aryléther éther cétones), poly(éther éther cétones), poly(éthylène téréphtalate), polyvalérates, cellulose de carboxyméthyl, cellulose, viscose, triacétates de viscose, nitrates de cellulose, acétates de cellulose, cellulose de hydroxyéthyle, butyrates de cellulose, acetate-butyrates de cellulose, copolymères d'éthyle-vinyle acétate, polysulfones, résines époxy, résines ABS, gommes EPDM, silicones, polysiloxanes, polydiméthyl siloxanes, halogènes de polyvinyle et copolymères, éthers de cellulose, triacétates de cellulose, chitosane et copolymères et/ ou mélanges de ceux-ci.

10. Méthode selon une revendication précédentes, où dans la solution de paclitaxel dans le diméthylsulfoxyde, choisi parmi le groupe comprenant: abciximab, acemétacine, acétyle vismione A, aclarubine, ademéthionine, adriamycine, aescine, afromosone, akagerine, aldesleukine, amiodarone, aminoglutéthimide, amsacrine, anakinra, anastrozole, anemonine, anopterine, antimycosiques, médicament anticoagulant, apocymarine, argatrobane, aristolactame-Ali, acide aristolochique, ascomycine, asparaginase, aspirine, atorvastatine, auranofine, azathioprine, azithromycine, baccatine, bafilomycine, basiliximab, bendamustine, benzocaïne, berbérine, betuline, acide betulinique, bilobol" bisparthenolidine, bléomycine, brombrestatine, acides boswelliques et leurs dérivés, bruceanoles A, B et C, bryophylline A, busulfan, antithrombine, bivalirudine, cadhérine, camptothécine, capecitabine, acide acétique o-carbamoyl-phénoxy, carboplatine, carmustine, celecoxib, cepharanthine, cerivastatine, inhibiteur de la CETP, chlorambucil, chloroquine phospate, cicutoxine, ciprofloxacine, cisplatine, cladribine, clarithromycine, colchicine, concanymycine, coumadine, natriurétique de type C (CNP), cudraisoflavone, curcumine, cyclophosphamide, cyclosporine A, cytarabine, dacarbazine, daclizumab, dactinomycine, dapsone, daunorubicine, diclofénac, 1,11-diméthoxycanthin-6-on, docétaxel, doxorubicine, dunaimycine, épirubicine, épothilones A et B, erythromycine, estramustine, étoboside, everolimus, filgrastime, fluoroblastine, fluvastatine, fludarabine, fludarabine-5'-dihydrogènophosphate, folimycine, fluorouracil, fosfestrol, gemcitabine, ghalakinoside, ginkgol, acide ginkgolique, glykoside 1a, 4-hydroxyoxycyclophosphamide, idarubicine, ifosfamide, josamycine, lapachone, lovastatine, lomustine, melphalan, midecamycine, mitoxantrone, nimustine, pitavastatine, pravastatine, procarbazine, mitomycine, méthotrexate, cladribine, mercaptopurine, thioguanine, oxaliplatine, irinotécan, topotécan, hydroxycarbamide, miltéfosine, pentostatine, pegaspargase, exémestane, létrozole, formestane, mitoxantrone, mycophénolate mofétil, ß-lapachone, podophyllotoxine, 2-ethylhydrazide de l'acide podophyllique, molgramostime (rhuGM-CSF), peginterféron α-2b, lénograstime (r-HuG-CSF), macrogol, sélectine (cytokine antagoniste), inhibiteurs de la cytokine, inhibiteur de la COX-2, angiopeptine, anticorps monoclonales inhibitant la prolifération des cellules musculaires, bFGF-antagonistes, probucol, prostaglandine, 1,11-diméthoxycanthin-6-on, 1-hydroxy-11-méthoxycanthin-6-on, scopoletine, colchicine, NO donneurs, pentaerythrityl tetranitrate et les sydnoimines, dérivés de S-Nitroso, tamoxifène, staurosporine, ß-estradiol, α-estradiol, estriol, estrone, ethinylestradiol, medroxy-progestérone, cipionates d'estradiol, benzoates d'estradiol, tranilast, kamebakaurin et autres terpénoïdes utilisés pour le traitement anticancéreux, vérapamil, inhibiteurs de tyrosine kinase (tyrphostine), paclitaxel est ses dérivés, 6-α-hydroxy-paclitaxel, taxotères mofebutazone, lonazolac, lidocaine, ketoprofène, acide méfénamique, piroxicam, meloxicam, penicillamine, hydroxychloroquine, aurothiomalate de sodium, oxaceprol, ß-sitosterine, myrtecaine, polidocanol, nonivamide, levomenthol, ellipticine, D-24851 (Calbiochem), colcemide, cytochalasine A-E, indanocine, nocodazole, bacitracine, antagonistes du récepteur de ka vitronectine, azélastine, stimulateur de la guanidyl cyclase, inhibiteur tissulaire des protéinases métalliques 1 et 2, acides nucléiques libres, acides nucléiques incorporés dans des transmetteurs de virus, fragments de DNA et RNA, inhibiteur activateur plasminogène 1, inhibiteur activateur plasminogène 2, oligonucléotides antisens, inhibiteurs VEGF, IGF-1, substances actives provenant du groupe des antibiotiques, céfadroxil, céfazoline, céfaclor, céfoxitine, tobramycine, gentamycine, pénicillines, dicloxacilline, oxacilline, sulfonamides, metronidazol, enoxaparine, héparine, hirudine, PPACK, protamine, prourokinase, streptokinase, wafarine, urokinase ; vasodilateurs, dipyridamol, trapidil, nitroprusside, antagonistes de PDGF, triazolopyrimidine, seramine, inhibiteurs de l'ACE, captopril, cilazapril, lisinopril, enalapril, losartan, inhibiteurs de la thioprotease, prostacycline, vapiprost, interféron α, ß et γ, antagonistes de l'histamine, bloqueurs de la sérotonine, inhibiteurs d'apoptose, régulateurs de l'apoptose, halofuginone, nifédipine, tocophérol, tranilast, molsidomine, polyphénoles du thé, épicatéchine gallate, épigallocatéchine gallate, leflunomide, etanercept, sulfasalazine, étpopside, dicloxacilline, tetracycline, triamcinolone, mutamycin, procainamde, acide rétinoïque, quinidine, disopyramide, flecainide, propafénone, sotalol; stéroïdes produites en manière naturelle et synthétique, inotodiol, maquiroside A, ghalakinoside, mansonine, strebloside, hydrocortisone, bétaméthasone, déxaméthason, substances non-stéroïdiens (NSAIDS), fénoprofène, ibuprofène, indométhacine, naproxène, phenylbutazone, agents antiviraux, acyclovir, ganciclovir zidovudine; clotrimazol, flucytosine, griséofulvine, kétoconazole, miconazole, nystatine, terbinafine ; agents antiprotozoaires, chloroquine, mefloquine, quinine, terpénoïdes naturelles, hippocaesculin, barringtogenol-C21-angelate, 14-déhydro-agrostistachin, agroskerine, agrostistachine, 17-hydroxy-agrostistachine, ovatodiolides, acide 4,7-oxycycloanisomelique, baccharinoides B1, B2, B3 et B7, tubeimoside, bruceanoles C, bruceantinosides C, yadanziosides N et P, isodéoxyelephantopine, tomenphantopine A et B, coronarine A, B, C et D, acide ursolique, acide hyptatique A, iso-iridogermanal, maytenfoliol, effusantine A, excisanin A et B, longikaurin B, sculponéatin C, kamebaunin, leukamenin A et B, 13,18-déhydro-6-alpha-senecioyloxychaparrin, taxamairin A et B, regenilol, triptolide ; cymarine, hydroxy anopterine, anémonine, proto-anémonine, chloride de cheliburine, sinococuline A et B, dihydronitidine, chloride de nitidine, 12-beta-hydroxy-pregnadiène-3,20-dione, helenalin, indicin, indicin-N-oxide, lasiocarpine, inotodiol, podophyllotoxine, justicidine A et B, larréatin, malloterin, mallotochromanol, isobutyryl-mallotochromanol, maquiroside A, marchantine A, maytansine, lycoridicine, margetine, pancratistatine, liriodenine, bisparthenolidine, oxoushimsunine, periplocoside A, acide usolique, deoxy-psorospermine, psycorubine, ricine A, sanguinarine, acide Manwuweiz (Manwuweizsäure), méthylsorbifolin, sphatheliachromes, stizophylline, dihydrousambaraensin, hydroxyusambarine, strychnopentamine, strychnophylline, usambarine, usambarensine, liriodenine, daphnoretine, lariciresinol, methoxy-lariciresinol, syringaresinol, sirolimus (rapamycine), somatostatine, tacrolimus, roxithromycine, troléandomycine, simvastatine, rosuvastatine, vinblastine, vincristine, vindesine, teniposide, vinorelbine, tropfofoamide, treosulfane, tremozolomide, thiotépa, trétinoïne, spiramycine, umbelliferone, déacetyl-vismione A, vismione A et B et zéorine.
